# EUROPEAN PATENT SPECIFICATION

(11) **EP 1 423 124 B1**
(45) Date of publication and mention of the grant of the patent: **08.08.2007**
(21) Application number: 02749634.8
(22) Date of filing: 05.07.2002
(51) Int. Cl.: A61K 31/5513, A61K 31/7012, A61K 47/12, A61K 47/26, A61K 9/19, A61P 25/18, A61P 25/28

(54) **LYOPHILIZED FORMULATION COMPRISING OLANZAPINE**
LYOPHILISIERTE FORMULIERUNG ENTHALTEND OLANZAPIN
FORMULATION LYOPHILISEE D'OLANZAPINE

(30) Priority: 20.07.2001 US 306829 P; 07.06.2002 US 386474 P
(43) Date of publication of application: 02.06.2004
(73) Proprietor: ELI LILLY AND COMPANY, Indianapolis, Indiana 46285 (US)
(72) Inventor: DEKEMPER, Kurt, Douglas, Franklin, IN 46131 (US); FITES, Alan, Lee, Martinsville, IN 46151 (US); NAIL, Steven, L., West Lafayette, IN 47906 (US)
(74) Representative: Suarez-Miles, Ana Sanchiz
(86) International application number: PCT/US2002/019799
(87) International publication number: WO 2003/007912

(56) References cited:
- EP-A- 0 421 297
- US-A- 5 457 101

## Description

This invention provides an amorphous, lyophilized parenteral formulation of 2-methyl-4-(4-methyl-1-piperazinyl)-10H-thieno[2,3-b][1,5] benzodiazepine, hereinafter referred to as olanzapine.

Olanzapine has shown great promise in the treatment of psychotic patients. Certain formulations of olanzapine are known, as described in U.S. Patent Nos. 5,229,382, 5,457,101 and 5,919,485. The presently claimed invention provides an amorphous, lyophilized, parenteral formulation comprising olanzapine as an active ingredient intimately mixed with a solubilizer and lactose as a stabilizer.

Olanzapine is a potent thienobenzodiazepine atypical antipsychotic agent displaying nanomolar receptor affinity in vitro at serotonin 5-HT2A/2B/2C, 5-HT3, 5-HT6, dopamine D1/D2/D3/D4/D5, muscarininic cholinergic (m1-m5), alpha1-adrenergic, and histamine H1 receptors. Orally administered olanzapine is currently used to treat patients worldwide as a marketed product and over 5.6 million patients have been treated with olanzapine to date.

However, in some cases, these patients are difficult to treat because of their presenting clinical state which can be agitated (overactive, chaotic and/or negative). Furthermore, other underlying conditions besides psychosis may also cause patients to become agitated (such as but not limited to post surgical delirium, behavioral disturbances associated with mental retardation, mood disorders, adjustment disorders, personality disorders, anxiety disorders).

Parenteral administration of antipsychotics is favored for the control of agitation where a rapid onset of action is desirable or when patients are unable to comply with oral preparations. Parenteral pharmacotherapy is clinically appropriate when the level of hostility, excitement, uncooperativeness or lack of impulse control is such that the potential exists for harm to self or others, or for the destruction of property.

The currently available intramuscular (IM) formulations of typical antipsychotics have significant safety and efficacy limitations including acute dystonias (sustained contraction of the muscles of the head, neck, or upper limbs), akathesia (persistent motor restlessness and muscle tightness), ECG abnormalities such as prolongation of the QTc interval, excessive sedation, and neuroleptic malignant syndrome (rigidity, fever, a fluctuating level of consciousness, autonomic instability, and elevated muscle enzymes).

Olanzapine is a highly potent compound which is efficacious at small doses. In light of the potent nature of olanzapine, consistent dose uniformity is imperative. Furthermore, olanzapine is metastable and subject to hydrolysis, particularly in solution or moist environments, at room temperature or even under refrigeration. In addition, olanzapine is relatively insoluble in parenteral diluents suitable for injection.

Lyophilized products are often difficult to prepare with a uniform, solid plug that doesn't degrade, adhere to the walls of the vial and stopper or leave behind product when reconstituted. Undissolved product is particularly problematic when dealing with a potent compound, such as olanzapine, because of potential dosing variation which could occur if the active agent isn't completely dissolved when reconstituted. Thus, there is a need for a stable, uniform, readily reconstitutable, rapid acting injectable formulation of olanzapine with a relatively long shelf life, suitable for use in difficult to treat, agitated patients. Such formulation, preferably, is isotonic and reconstitutable in a small injection volume.

Applicants have created an injectable, amorphous, lyophilized formulation which combines olanzapine with a suitable solubilizer and suitable stabilizer and provides the desired physical and chemical stability, ease of reconstitution, uniformity, long shelf life and rapid action needed to treat agitated patients. Applicants have found that the injectable lyophilized, amorphous formulation of olanzapine provides superior therapy for agitated patients. The formulation is preferably useful for the rapid control of agitation and disturbed behaviors associated with agitation associated with schizophrenia, bipolar manic and mixed states, dementia and associated disorders, and neurodegenerative disorders. As used herein, the term "neurodegenerative disorders" means conditions associated with retrogressive neuronal pathological change leading to loss of central nervous system function.

The term "treating" as used herein includes prophylaxis of the named condition or amelioration or elimination of the condition once it has been established.

The term "lyophilized formulation" means a freeze-dried formulation prepared by the processes known in the art and comprising as essential ingredients olanzapine, a solubilizer and lactose as a stabilizer.

By "rapid acting" is meant a parenteral formulation that is effective in alleviating or substantially reducing agitation in an agitated patient in from about one minute to about twelve hours after injection, preferably in from about five minutes to about ten hours after injection, most preferably in from about fifteen minutes to about two hours after injection.

The term "agitation" or "agitated patient" as used herein means excessive motor or verbal activity that is usually nonproductive and repetitious. Its core psychiatric symptoms include hostility, tension, excitement, uncooperativeness and poor impulse control.

Agitation may occur with many disorders. It is a common component of psychotic disorders, schizophrenia, bipolar disorder (both manic and mixed states), dementia and associated disorders and neurodegenerative disorders. Its phenomenology is remarkably similar across disease states and its clinical description has been well characterized.

The Diagnostic and Statistical Manual 4^{th} Edition (DSM IV) defines psychomotor agitation as "excessive motor activity...that is usually nonproductive and repetitious" ( DSM IV, Fourth Edition, Washington DC, American Psychiatric Association, p 763, 1994). Examples of motor manifestations of agitation are hyperactivity, assaultiveness, physical destructiveness and threatening gestures. Other authors describe verbal forms of agitation as excessive verbal or vocal expression which include vocal outbursts, threatening language, verbal abuse and excessive verbalizations of distress (Cohen-Mansfiels and Billing, J Am Geriatr Soc 34:711-721, 1986; Lanz and Marin, J Geriatr Psychiatry Neurol 9:107-119, 1996; Mintzer and Brawman-Mintzer, J Clin Psychiatry 57(s):55-63,1996; Fugate et al., Arch Phys Med Rehabil 78:917-923, 1996; Lindenmayer, J Clin Psychiatry 61(suppl 14):5-10,2000).

In the instant invention, the term "agitation" encompasses agitation associated with any recognized form of psychopathology, preferably, agitation associated with schizophrenia, bipolar manic and mixed states, dementia and associated disorders and neurodegenerative disorders. More specifically, the term "agitation" includes agitation associated with Delirium, Dementia and Amnesiac and Other Cognitive Disorders (DSM IV pp 123- 164), Mental Disorders Due to a General Medical Condition (DSM IV pp 165-174), Substance Related Disorders (DSM IV pp 175- 272), Schizophrenia and Other Psychotic Disorders (DSM IV 273-316), Mood Disorders (DSM IV pp 317-392), Anxiety Disorders (DSM IV pp 393674 444), Adjustment Disorders (DSM IV pp 623-628), or Personality Disorders (DSM IV pp 629- 674).

Olanzapine Form II is the most stable anhydrous form of olanzapine known and is therefore important for the commercial development of pharmaceutically elegant formulations. However, Form II is unsuitable for lyophilized formulations because it is does not readily dissolve in conventional parenteral diluents. In accordance with this invention, Applicants have discovered, surprisingly, that amorphous olanzapine, particularly amorphous olanzapine tartrate, is preferred for a stable, rapid acting, readily reconstitutable, lyophilized olanzapine formulation with a consistently reproducible dose.

The present invention provides a stable, amorphous, lyophilized, parenteral formulation comprising olanzapine as an active ingredient, a solubilizer and lactose as a stabilizer. Such formulation is particularly useful for treating agitation. Yet another embodiment of the invention is the use of the formulation for the manufacture of a medicament for the treatment of agitation. In still another preferred embodiment, is a pharmaceutical formulation for use in treating agitation in a human, comprising an active ingredient for treating agitation, a solubilizer and lactose as a stabilizer, characterized in that said active ingredient is olanzapine.

The present invention further provides a formulation which may be administered via parenteral, intramuscular injection.

Another preferred embodiment of the formulation of the invention, is one where the olanzapine is a tartrate salt.

Preferably, the solubilizer of the formulation is tartaric acid and the stabilizer is lactose. In an additionally preferred embodiment, the lactose is lactose monohydrate.

Preferred is a formulation comprising from 1 to 20 mg/ml olanzapine, from 20 to 50 mg/ml of lactose monohydrate and from 0.35 to 10 mg/ml of tartaric acid.

Particularly preferred is a formulation comprising from 1 to 20 mg/ml olanzapine, from 22.5 to 50 mg/ml of lactose monohydrate and from 0.35 to 10 mg/ml of tartaric acid.

Additionally preferred is a formulation comprising 5 mg/ml of olanzapine, 1.7 mg/ml of tartaric acid and 25 mg/ml of lactose monohydrate.

In another preferred embodiment of the invention, the amount of olanzapine in the formulation is selected from 1, 2.5, 5, 7.5, 10, 15, 20 mg/ml.

An especially preferred embodiment of the invention is one comprising 20 mg olanzapine, 7 mg tartaric acid and 100 mg lactose.

In a further embodiment of the invention, Applicants have found the formulation of the invention particularly useful for treating agitation associated with disorders selected from psychotic disorders, schizophrenia, bipolar disorder (both manic and mixed states), dementia and associated disorders, and neurodegenerative disorders.

Especially preferred is the formulation when used to treat agitation associated with disorders selected from Delirium, Dementia and Amnesiac and Other Cognitive Disorders (DSM IV pp 123- 164), Mental Disorders Due to a General Medical Condition (DSM IV pp 165-174), Substance Related Disorders (DSM IV pp 175- 272), Schizophrenia and Other Psychotic Disorders (DSM IV 273-316), Mood Disorders (DSM IV pp 317-392), Anxiety Disorders (DSM IV pp 393674 444) Adjustment Disorders (DSM IV pp 623-628), and Personality Disorders (DSM IV pp 629- 674).

The term "amorphous" means a physical state having no crystal lattice structure and may be verified by X-ray diffraction, solid-state NMR (SSNMR) and other supportive means, such as observation with a polarized light microscope and Differential Scanning Calorimetry (DSC).

X-ray powder diffraction may be used to establish that the lyophilized formulation is in an amorphous state. The X-ray powder diffraction (XRD) patterns for the lyophilized olanzapine formulation is obtained on a Siemens D5000 X-ray powder diffractometer, equipped with a CuKα source (λ = 1.54056 Å) and a Kevex solid-state detector, operating at minimally 35 kV and 30 mA. The sample is scanned between 4° and 35° in 2θ, with a maximum scanning rate of 0.05° in 2θ per second. A typical XRD pattern for the amorphous, lyophilized olanzapine formulation is shown in Figure I

¹³C Cross polarization / magic angle spinning (CP/MAS) NMR (SSNMR) may be used to establish that olanzapine in the lyophilized formulation is stabilized in the amorphous state as a tartrate salt. ¹³C Cross polarization / magic angle spinning (CP/MAS) NMR (solid-state NMR or SSNMR) spectra are obtained using a Varian Unity Inova 400 MHz NMR spectrometer operating at a carbon frequency of 100.573 MHz and equipped with a complete solids accessory and either a Varian 5 mm VT CP/MAS or a Chemagnetics 7.5 mm T3 probe. Acquisition parameters are as follows: 90° proton r.f. pulse width 5.0 µs, contact time 2.0 ms, pulse repetition time 10 s, MAS frequency 7.0 kHz, spectral width 50 kHz, and acquisition time 50 ms. Chemical shifts are referenced to the methyl group of hexamethylbenzene (δ = 17.3 ppm) by sample replacement. Identification of olanzapine tartrate is based upon comparative chemical shift data collected on isostructural solvates of olanzapine hemi-tartrate using the same experimental procedures for SSNMR data collection as above. A typical SSNMR pattern which demonstrates that olanzapine is stabilized in the amorphous state as a tartrate salt is shown in figure II.

Most preferably, the amorphous olanzapine tartrate of the formulation will be free of crystalline forms of olanzapine and contain less than about 1.5% total related substances, wherein "related substances" refers to undesired chemical impurities and degradation products of olanzapine.

Lyophilized formulations containing alternative stabilizers were evaluated. Samples are assayed for potency and related substances after about two and three weeks storage at 5°C, 25°C, 40°C /75% relative humidity, and 50°C. Formulations containing lactose, surprisingly, have greater potency and show less degradation than formulations containing other stabilizers. Therefore, lactose is a surprising and important component of a stable, lyophilized formulation of olanzapine. Lactose monohydrate is preferred.

Suitable solubilizers include hydrochloric acid, phosphoric acid and organic acids such as, but not limited to, tartaric acid, citric acid, acetic acid, malic acid and fumaric acid. Tartaric acid is preferred for optimal solubility, dispersion, stability and freeze-dry characteristics.

Olanzapine is effective over a wide dosage range, the actual dose administered being dependent on the condition being treated. For example, in the treatment of adult humans, dosages of from about 0.25 to 50 mg, preferably from 1 to 30 mg, and most preferably 1 to 20 mg per day may be used. The specific dose of olanzapine administered according to this invention to obtain therapeutic effects will, of course, be determined by the particular circumstances surrounding the case, including the condition being treated. For treatment of agitated patients a dose range of from 1 to 20 mg/injection is suitable, preferably 2.5 to 12.5 mg/injection, most preferably 10mg/injection. Agitated patients may receive from one to three, preferably one, injection(s) per day. It may be appreciated that it may be necessary to make routine variations to the dosage depending on the age and condition of the patient.

A lyophilized formulation of the invention is a formulation comprising from 1 to 20 mg/ml olanzapine as an active ingredient (preferably from 1 to 10 mg/ml), from 22.5 to 50 mg/ml of lactose monohydrate and from 0.35 to 10 mg/ml of tartaric acid.

A preferred lyophilized formulation of the invention is a formulation comprising from 1 to 20 mg/ml olanzapine as an active ingredient (preferably from 1 to 10 mg/ml), from 20 to 50 mg/ml of lactose monohydrate and from 0.35 to 10 mg/ml of tartaric acid. An excess of tartaric acid relative to olanzapine, preferably in the range of 1.67/l to 0.5/l weight/weight of olanzapine/tartaric acid, is necessary to maintain the olanzapine solubility. Especially preferred is a lyophilized formulation comprising 5 mg/ml mg of olanzapine as an effective amount of the active ingredient, 1.7 mg/ml of tartaric acid and 25 mg/ml of lactose monohydrate. Further, such formulation preferably will contain not more than about 0.1% to about 1.5% total related substances, more preferably not more than about 1.0% total related substances. Additionally, such formulations preferably will contain not more than about 4% moisture, more preferably less than about 0.1% moisture.

The formulation is preferably reconstitutable in from about 1.1 to about 2.2 ml of diluent, preferably 2 ml, to obtain solutions having concentrations of approximately 5 and 10 mg/ml, and can be dissolved in a pharmaceutically acceptable carrier, such as sterile water for injection (WFI), sterile water optionally containing saline and/or sterile water containing sugars. For example, for intramuscular injection, the lyophilized plug may be dissolved in 2 ml of WFI.

A study of the lyophilized formulation stored under accelerated stability conditions for almost a year show pharmaceutically acceptable potency and stability. Samples are stored at 25°C, 40°C and 75% relative humidity, and 50°C and are assayed for potency and percent related substances at 39 days, 77 days, 109 days 202 days and 343 days.

The materials for the present invention can be purchased or prepared by a variety of procedures well known to those of ordinary skill in the art. Olanzapine can be prepared as described by Chakrabarti in U.S. Patent No 5,229,382 ('382).

The lyophilized, amorphous formulation of the present invention can be prepared by freeze drying a solution containing technical grade olanzapine, lactose and a solubilizer, in a high vacuum for sublimating water.

Preferably, the lyophilization solution is prepared by dissolving about 3.5 mg/ml of tartaric acid (solubilizer), and about 50 mg/ml lactose, in a suitable solvent, preferably water or Water For Injection (WFI). The solubilizer and lactose are mixed and stirred until they dissolve and the solution is preferably cooled to a temperature of about 2 °C to about 25 °C, preferably about 6 °C to about 12 °C, prior to the addition of olanzapine. Alternately, lactose may be added after the addition of olanzapine. From about 1mg/ml to about 10mg/ml olanzapine is added, preferably about 5mg/ml. The pH is checked and adjusted, if necessary. The pH of the solution should be in the range of from about 5.2 to about 6.0, preferably 5.5 to 5.7. The pH may be adjusted with HC1 or NaOH. The solution is then brought to the proper volume with WFI. The initial volume of WFI should be at least 90% of the total volume to achieve acceptable dissolution of the olanzapine.

Since hydrolysis is the main path of degradation, the holding time and temperature of the solution prior to lyophilization are critical. It is recommended that the lyophilization solution be refrigerated at from about 2 to 12 °C, preferably less than 10 °C, prior to filling. The solution should be filled and lyophilization initiated within from about 1 to about 48 hours of solution manufacture, preferably within 24 hours.

A secondary drying cycle, sufficient to reduce moisture in the final product to less than 0.6% is preferred to minimize olanzapine degradation and provide optimum shelf life. The lyophilized plug readily dissolves when reconstituted with a pharmaceutically acceptable diluent.

If desired, before lyophilization the lyophilization solution may be subjected to a filtration process. The filtration process may include, for example, a sterilizing filtration of the lyophilization solution to eliminate microorganisms or other contaminating matter.

If desired, before lyophilization, the lyophilization solution may be subjected to a distributing process. The distributing process includes, for example in the case of vial lyophilizations, a process for distributing a suitable volume of the lyophilization solution before lyophilization into vials, taking the concentration of olanzapine into consideration in order that vial products carry a desired amount of olanzapine.

The lyophilized composition is prepared by a sequential cooling and heating process. Preferably, the process for preparing a lyophilized composition comprises the sequential steps of:
(a) cooling the lyophilization solution to a temperature below -35 °C for at least one hour;
(b) heating the product of step (a) to a temperature of at least -22°C for at least eight hours under subatmospheric pressure to remove water;
(c) heating the product of step (b) to a temperature of at least 30 °C, preferably from about 30 °C to about 40 °C, for a time sufficient to remove water from the aqueous solvent and yield a solid lyophilized product, preferably at least six hours under subatmospheric pressure. Steps (b) and (c) are performed at a subatmospheric pressure of less than 125 mTorr, preferably from about 90 mTorr to about 115 mTorr.

Preferred parameters in the lyophilization process are those wherein olanzapine is frozen by cooling to less than -35 °C, preferably from about -35 °C to about -45°C. This cooling step is performed preferably over 1 to 4 hours. This process is herein after referred to as the "primary freezing process".

The frozen solution obtained in the primary freezing process is then warmed to a temperature in the range of from about -15°C to about -25°C, preferably at greater than or equal to -22°C, under subatmospheric pressure, preferably in the range of from about 90 mTorr to about 115mTorr. This warming step is preferably carried to completion in from 6 to 40 hours. This process is hereinafter referred to as the "primary drying process".

The composition obtained through the primary drying process is dried under a high vacuum by sublimating water according to methods known to those skilled in the art. Thus, a lyophilized preparation of the present invention is obtained. If desired, two step drying in which the temperature and the degree of vacuum are different may be performed for removing water, according to methods known to those skilled in the art. For example, the second drying step may be performed at temperatures of from about 30°C to about 40 °C, preferably from about 30°C to about 35 °C for at least six hours.

The lyophilization process removes most of the water originally present, but the final lyophilized product composition may contain residual free water, preferably less than 2.5%. Typically, the water content can range from about 0.1% to about 2.5% weight percent. More typically, the water content ranges from about 0.2% to about 0.6%.

A dose-concentrate configuration of the formulation of the invention is a sealed container holding an amount of lyophilized pharmaceutical formulation of the invention. The dose-concentrate configuration is prepared by placing lyophilized composition in a container (e.g., glass or plastic bottles, vials, ampoules, cartridges, syringes) in sufficient amount to treat at least one mammal. (As used herein, the term "mammal shall refer to the Mammilla class of higher vertebrates. The term "mammal" includes but is not limited to a human as well as related important veterinary species of mammals, domesticated quadripeds such as monkeys, dogs, cats, horses, sheep, pigs, goats and cows.)

The container preferably also contains an empty space of sufficient size to permit (i) addition of aqueous solvent plus (ii) additional space as necessary to permit agitation and effect complete solution of the lyophilized composition in the added aqueous solvent. The container may be equipped with a penetrable top, for example, a rubber seal, preferably a low moisture butyl rubber stopper, so that aqueous solvent may be added by penetrating the seal with a hypodermic needle and the concentrate subsequently removed by the same means. An example of a dose-concentrate configuration is a glass vial having a capacity of from about 5 to about 100 milliliters containing 1 to 150 milligrams of olanzapine in the lyophilized pharmaceutical formulation. The empty space above the solid composition has ample room for addition of a solvent such as sterile water for injection plus room to agitate the total contents. The addition of the aqueous solvent to the dose-concentrate configuration results in a liquid concentrate which may then be conveniently used to form unit dosages of liquid pharmaceutical formulation by removing the entire contents for immediate use as an intramuscular injection or for dilution for intravenous use. For intravenous use, the concentrate is added to an IV (intravenous) container containing a suitable aqueous solvent. Useful solvents are standard solutions for injection (e.g., 5% dextrose or sterile Water For Injection, etc.). Typical unit dosage IV bags are conventional glass or plastic containers having inlet and outlet means and having standard (e.g., 250 ml and 500 ml) capacities. The concentrated solution of lyophilized pharmaceutical formulation is added to the unit dose IV bag in an amount sufficient to achieve total dose of from about 1 to about 40 mg of olanzapine. Other pharmaceutically acceptable additive agents may be added to the lyophilized preparations of the present invention. Where the lyophilized preparation is to be used for injection, an isotonizing agent or a soothing agent or other additives may be added thereto.

Intramuscular injection is the preferred mode of delivery to the mammal being treated particularly in emergency situations. A specific example, is a 5 ml glass vial with a rubber stopper with low moisture hold-up and low vapor transmission rates, having a lyophilized pharmaceutical composition containing 10 mg of olanzapine, 3.5 mg of tartaric acid, and 50 mg of lactose monohydrate. Preferably the lyophilized pharmaceutical formulation of this invention is diluted with aqueous solvent suitable for injection and a liquid unit dosage form prepared for administration to a mammal. The rate of absorption of olanzapine is more rapid after parenteral administration, such as IM, than after oral administration of the same dose.

The efficacy of the lyophilized, amorphous, parenteral olanzapine formulation for the control of agitation is evaluated in a randomized, double-blind, placebo- and active comparator-controlled study in agitated patients, such as those, for example, with schizophrenia, bipolar mania, and dementia associated with neurodegenerative disorders. The patients represent a range of ages from young to elderly, and a range of clinical conditions involving both psychotic and nonpsychotic patients with moderate to severe agitation.

Alleviation of agitation is assessed by the use of a battery of measures, including but not limited to, the mean change from baseline in the Positive and Negative Syndrome Scale (PANSS) Excited Component (Kay et al., Schizophrenia Bulletin, 16, 537-545 1990). Additional efficacy measures include the Agitation-Calmness Evaluation Scale (ACES), the Corrigan Agitated Behavior Scale (Corrigan JD Journal of Clinical and Experimental Neuropsychology 11(2):262-77,1989) and the Cohen-Mansfield Agitation Inventory (Cohen-Mansfield et. Al., Journal of Clinical Psychiatry, 57(5):190-8, 1996; Cohen-Mansfield et., Int Psychogeriatrics 4:221-240, 1992; Cohen Mansfield et. Al., Gerontology, 36(3);150-8, 1990). The results of the studies support the efficacy of the lyophilized olanzapine formulation in treating agitation across different patient populations.

The ACES is designed to differentiate between the agitated, calm, and sleep states by utilizing a specially developed 9-point scale:
1 = Marked Agitation
2 = Moderate Agitation
3 = Mild Agitation
4 = Normal
5 = Mild Calmness
6 = Moderate Calmness
7 = Marked Calmness
8 = Deep Sleep
9 = Unarousable
Scores could range from a single score of 1 to 9.

The onset of action of the parenteral, lyophilized, amorphous olanzapine formulation is investigated at various time points ranging from 15 minutes to 2 hours following the first injection. The lyophilized olanzapine formulation demonstrated superior reduction in agitation on the PANSS Excited Component at the earliest time point (15 minutes) measured.

The following examples are provided for purposes of illustration.

### Example 1

Olanzapine for Injection, 10mg, Unit formula (mg/vial) Olanzapine 10 mg
Lactose Monohydrate 50 mg
Tartaric Acid 3.5 mg
WFI q.s. to 2 ml
HCl soln 10% and/or NaOH soln 10% q.s. for pH adjustment

### Preparation:

1. Dispense WFI into manufacturing container (approximately 75 to 90% of q.s. weight)
2. Add tartaric acid into water and stir until dissolved.
3. Add lactose monohydrate into manufacturing container and stir until dissolved.
4. Add olanzapine and stir until dissolved (5 to 60 minutes).
5. Adjust pH to 5.50 to 5.70 with 10% NaOH and/or 10% HCL solution as required.
6. Q.S. to final weight with WFI.
7. Check pH; readjust to 5.50 to 5.70 if necessary.
8. Filter solution through a 0.22 micron microbial retentive filter.
9. Fill to appropriate volume.
10. Freeze dry.

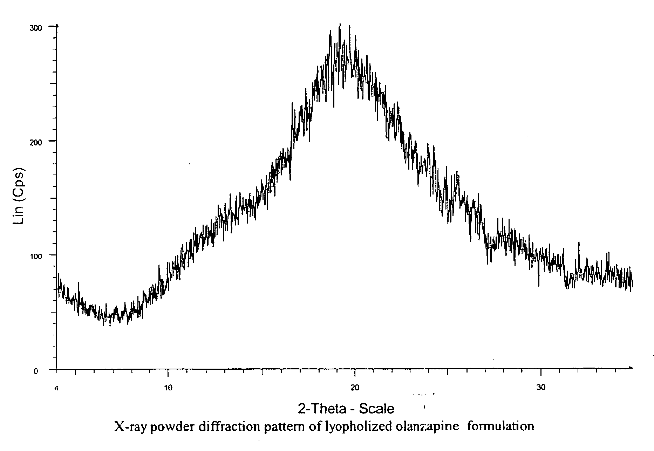

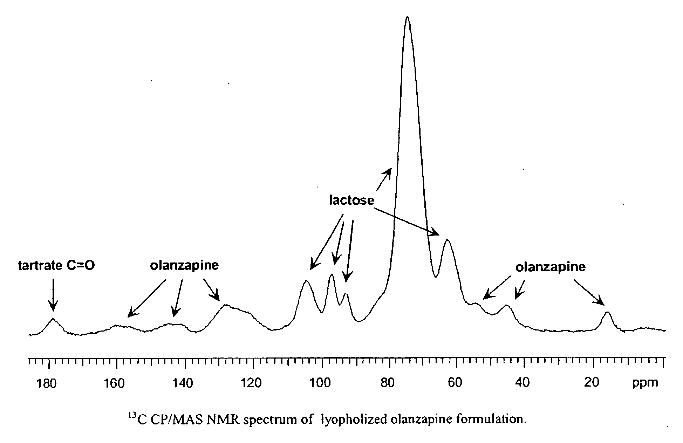

## Claims

1. A stable, amorphous, lyophilized, parenteral formulation comprising olanzapine as an active ingredient, a solubilizer and lactose as a stabilizer.

2. The formulation according to Claim 1 wherein the solubilizer is tartaric acid.

3. The formulation according to Claim 2 wherein the olanzapine is a tartrate salt.

4. The formulation according to Claim 2 wherein the lactose is lactose monohydrate.

5. The formulation according to Claim 4 comprising from 1 to 20 mg/mL olanzapine, from 20.0 to 50 mg/mL of lactose monohydrate and from 0.35 to 10 mg/mL of tartaric acid.

6. The formulation according to Claim 4 comprising from 1 to 20 mg/ml olanzapine, from 22.5 to 50 mg/ml of lactose monohydrate and from 0.35 to 10 mg/ml of tartaric acid.

7. The formulation according to Claim 4 comprising 5 mg/ml of olanzapine, 1.7 mg/ml of tartaric acid and 25 mg/ml of lactose monohydrate.

8. The formulation according to Claim 4 wherein the amount of olanzapine is selected from 1, 2.5, 5, 7.5, 10, 15, 20 mg/ml.

9. The formulation according to Claim 4 comprising 20 mg olanzapine, 7 mg tartaric acid and 100 mg lactose.

10. The formulation according to any one of Claims 1 to 9 for use in treating agitation.

11. The formulation according to Claim 10 wherein the agitation is associated with disorders selected from psychotic disorders, schizophrenia, bipolar disorder (both manic and mixed states), dementia and associated disorders, and neurodegenerative disorders.

12. The formulation according to Claim 10 wherein the agitation is associated with disorders selected from Delirium, Dementia and Amnesiac and Other Cognitive Disorders (DSM IV pp 123- 164), Mental Disorders Due to a General Medical Condition (DSM IV pp 165-174), Substance Related Disorders (DSM IV pp 175- 272), Schizophrenia and Other Psychotic Disorders (DSM IV 273-316), Mood Disorders (DSM IV pp 317-392), Anxiety Disorders (DSM IV pp 393674 444) Adjustment Disorders (DSM IV pp 623-628), and Personality Disorders (DSM IV pp 629- 674).

13. The use of a formulation as claimed in any one of Claims 1 to 9 for the manufacture of a medicament for the treatment of agitation.

14. A formulation obtainable by a process which comprises, lyophilization of a solution comprising olanzapine, a solubilizer, and lactose.

15. The formulation according to claim 14 wherein the solution is an aqueous solution.

16. The formulation according to claim 14 or 15 wherein the solubilizer is tartaric acid.

17. The formulation according to claim 16 wherein the formulation is placed in a container in sufficient amount to treat one mammal.

18. The formulation according to claim 17 wherein the amount of olanzapine is from 0.25 to 50 mg.

19. The formulation according to claim 17 wherein the amount of olanzapine is from 1 to 30 mg.

20. A process for preparing a formulation which comprises, lyophilization of a solution comprising olanzapine, a solubilizer, and lactose.

21. The process according to claim 20 wherein the solution is an aqueous solution.

22. The process according to claim 20 or 21 wherein the solubilizer is tartaric acid.

23. The process according to claim 22 wherein the formulation is placed in a container in sufficient amount to treat one mammal.

## Patentansprüche

1. Stabile, amorphe, lyophilisierte, parenterale Formulierung, umfassend Olanzapin als einen Wirkstoff, einen Solubilisator und Lactose als einen Stabilisator.

2. Formulierung nach Anspruch 1, worin der Solubilisator Weinsäure ist.

3. Formulierung nach Anspruch 2, worin das Olanzapin ein Tartratsalz ist.

4. Formulierung nach Anspruch 2, worin die Lactose Lactosemonohydrat ist.

5. Formulierung nach Anspruch 4, die 1 bis 20 mg/ml Olanzapin, 20,0 bis 50 mg/ml Lactosemonohydrat und 0,35 bis 10 mg/ml Weinsäure umfasst.

6. Formulierung nach Anspruch 4, die 1 bis 20 mg/ml Olanzapin, 22,5 bis 50 mg/ml Lactosemonoyhdrat und 0,35 bis 10 mg/ml Weinsäure umfasst.

7. Formulierung nach Anspruch 4, die 5 mg/ml Olanzapin, 1,7 mg/ml Weinsäure und 25 mg/ml Lactosemonohydrat umfasst.

8. Formulierung nach Anspruch 4, worin die Menge an Olanzapin ausgewählt ist aus 1, 2,5, 5, 7,5, 10, 15 und 20 mg/ml.

9. Formulierung nach Anspruch 4, die 20 mg Olanzapin, 7 mg Weinsäure und 100 mg Lactose umfasst.

10. Formulierung nach einem der Ansprüche 1 bis 9 zur Verwendung für die Behandlung einer Agitation.

11. Formulierung nach Anspruch 10, worin die Agitation assoziiert ist mit Störungen, die ausgewählt sind aus psychotischen Störungen, Schizophrenie, bipolaren Störungen - sowohl manischen als auch gemischten Zuständen -, Demenz und assoziierten Störungen, sowie neurodegenerativen Störungen.

12. Formulierung nach Anspruch 10, worin die Agitation assoziiert ist mit Störungen, die ausgewählt sind aus Delirium, Demenz und Amnesie und sonstigen Kognitionsstörungen (DSM IV, Seiten 123 bis 164), Mentalstörungen infolge eines allgemeinen medizinischen Zustands (DSM IV, Seiten 165 bis 174), substanzbezogenen Störungen (DSM IV, Seiten 175 bis 272), Schizophrenie und sonstigen psychotischen Störungen (DSM IV, Seiten 273 bis 316), Gemütsstörungen (DSM IV, Seiten 317 bis 392), Angststörungen (DSM IV, Seiten 393 bis 444), Einstellungsstörungen (DSM IV, Seiten 623 bis 628) und Persönlichkeitsstörungen (DSM IV, Seiten 629 bis 674).

13. Verwendung einer Formulierung nach einem der Ansprüche 1 bis 9 zur Herstellung eines Arzneimittels für die Behandlung von Agitation.

14. Formulierung, erhältlich nach einem Verfahren, das umfasst eine Lyophilisation einer Lösung, die Olanzapin, einen Solubilisator und Lactose umfasst.

15. Formulierung nach Anspruch 14, worin die Lösung eine wässrige Lösung ist.

16. Formulierung nach Anspruch 14 oder 15, worin der Solubilisator Weinsäure ist.

17. Formulierung nach Anspruch 16, worin die Formulierung in einem Behältnis in einer Menge angeordnet ist, die für eine Behandlung eines Säugers ausreicht.

18. Formulierung nach Anspruch 17, worin die Menge an Olanzapin 0,25 bis 50 mg beträgt.

19. Formulierung nach Anspruch 17, worin die Menge an Olanzapin 1 bis 30 mg beträgt.

20. Verfahren zur Herstellung einer Formulierung, umfassend eine Lyophilisation einer Lösung, die Olanzapin, einen Solubilisator und Lactose umfasst.

21. Verfahren nach Anspruch 20, worin die Lösung eine wässrige Lösung ist.

22. Verfahren nach Anspruch 20 oder 21, worin der Solubilisator Weinsäure ist.

23. Verfahren nach Anspruch 22, worin die Formulierung in einem Behältnis in einer Menge angeordnet ist, die zur Behandlung eines Säugers ausreicht.

## Revendications

1. Formulation stable, amorphe, lyophilisée, parentérale, comprenant l'olanzapine, en tant qu'ingrédient actif, un solubilisant et le lactose en tant que stabilisant.

2. Formulation selon la revendication 1, dans laquelle le solubilisant est l'acide tartrique.

3. Formulation selon la revendication 2, dans laquelle l'olanzapine est un sel tartrate.

4. Formulation selon la revendication 2, dans laquelle le lactose est le lactose monohydraté.

5. Formulation selon la revendication 4, comprenant de 1 à 20 mg/ml d'olanzapine, de 20,0 à 50 mg/ml de lactose monohydraté, et de 0,35 à 10 mg/ml d'acide tartrique.

6. Formulation selon la revendication 4, comprenant de 1 à 20 mg/ml d'olanzapine, de 22,5 à 50 mg/ml de lactose monohydraté, et de 0,35 à 10 mg/ml d'acide tartrique.

7. Formulation selon la revendication 4, comprenant 5 mg/ml d'olanzapine, 1,7 mg/ml d'acide tartrique et 25 mg/ml de lactose monohydraté.

8. Formulation selon la revendication 4, dans laquelle la quantité d'olanzapine est choisie parmi 1; 2,5; 5; 7,5; 10; 15; 20 mg/ml.

9. Formulation selon la revendication 4, comprenant 20 mg d'olanzapine, 7 mg d'acide tartrique et 100 mg de lactose.

10. Formulation selon l'une quelconque des revendications 1 à 9, destinée à une utilisation dans le traitement de l'agitation.

11. Formulation selon la revendication 10, dans laquelle l'agitation est associée à des troubles choisis parmi les troubles psychotiques, la schizophrénie, le trouble bipolaire (à la fois les états maniaques et mixtes), la démence et les troubles associés, et les troubles neurodégénératifs.

12. Formulation selon la revendication 10, dans laquelle l'agitation est associée à des troubles choisis parmi le délire, la démence et l'amnésie, et autres troubles cognitifs (DSM IV pages 123-164), les troubles mentaux dus à un état médical général (DSM IV pages 165-174), les troubles liés aux substances psychotropes (DSM IV pages 175-272), la schizophrénie et autres troubles psychotiques (DSM IV pages 273-316), les troubles de l'humeur (DSM IV pages 317-392), les troubles de l'angoisse (DSM IV pages 393-444), les troubles de l'adaptation (DSM IV pages 623-628), et les troubles de la personnalité (DSM IV pages 629-674).

13. Utilisation d'une formulation selon l'une quelconque des revendications 1 à 9, pour la fabrication d'un médicament destiné au traitement de l'agitation.

14. Formulation pouvant être obtenue à l'aide d'un procédé qui comprend la lyophilisation d'une solution comprenant de l'olanzapine, un solubilisant et du lactose.

15. Formulation selon la revendication 14, dans laquelle la solution est une solution aqueuse.

16. Formulation selon la revendication 14 ou 15, dans laquelle le solubilisant est l'acide tartrique.

17. Formulation selon la revendication 16, dans laquelle la formulation est placée dans un récipient en une quantité suffisante pour traiter un mammifère.

18. Formulation selon la revendication 17, dans laquelle la quantité d'olanzapine est de 0,25 à 50 mg.

19. Formulation selon la revendication 17, dans laquelle la quantité d'olanzapine est de 1 à 30 mg

20. Procédé de préparation d'une formulation qui comprend la lyophilisation d'une solution comprenant de l'olanzapine, un solubilisant et du lactose.

21. Procédé selon la revendication 20, dans lequel la solution est une solution aqueuse.

22. Procédé selon la revendication 20 ou 21, dans lequel le solubilisant est l'acide tartrique.

23. Procédé selon la revendication 22, dans lequel la formulation est placée dans un récipient en une quantité suffisante pour traiter un mammifère.
